# FASCICULE DE BREVET EUROPEEN

(11) **EP 3 584 006 B1**
(45) Date de publication et mention de la délivrance du brevet: **09.10.2024**
(21) Numéro de dépôt: 19191127.0
(22) Date de dépôt: 06.01.2015
(51) Int. Cl.: B01J 13/00, C08F 220/28, A61K 8/02, A61K 8/04, A61K 8/81, A61Q 1/00, A61Q 19/00

(54) **PRODUIT COSMETIQUE OU PHARMACEUTIQUE CONTENANT DES MICROGELS DE POLY(ETHYLENE GLYCOL-METHACRYLATE)**
KOSMETISCHES ODER PHARMAZEUTISCHES PRODUKT, DAS MIKROGELE AUS POLY(ETHYLENGLYCOL-METHACRYLAT) ENTHÄLT
COSMETIC OR PHARMACEUTICAL PRODUCT CONTAINING POLY(ETHYLENE GLYCOL METHACRYLATE) MICROGELS

(43) Date de publication de la demande: 25.12.2019
(62) Demande divisionnaire de: 15703307.7
(73) Titulaire: LVMH Recherche, 45800 Saint Jean de Braye (FR); Centre National de la Recherche Scientifique (C.N.R.S.), 75016 Paris (FR); Université de Pau et des Pays de l'Adour, 64012 Pau Cedex (FR)
(72) Inventeur: TRANCHANT, Jean-François, 45760 Marigny Les Usages (FR); GOMBART, Emilie, 45100 Orléans (FR); BILLON, Laurent, 64110 Saint Faust (FR); SAVE, Maud, 65250 Escala (FR); BOULARAS, Mohamed, 78130 Les Mureaux (FR)
(74) Mandataire: Cabinet Beau de Loménie

(56) Documents cités:
- WO-A1-2006/037901
- WO-A1-2006/037907
- MOHAMED BOULARAS ET AL: "Design of Smart Oligo(ethylene glycol)-Based Biocompatible Hybrid Microgels Loaded with Magnetic Nanoparticles", MACROMOLECULAR RAPID COMMUNICATIONS, vol. 36, no. 1, 25 November 2014 (2014-11-25), pages 79 - 83, XP055212999, ISSN: 1022-1336, DOI: 10.1002/marc.201400578
- MOHAMED BOULARAS ET AL: "Supporting informations, Design of Smart Oligo(ethylene glycol)-Based Biocompatible Hybrid Microgels Loaded with Magnetic Nanoparticles", MACROMOLECULAR RAPID COMMUNICATIONS, vol. 36, no. 1, 25 November 2014 (2014-11-25), pages 79 - 83, XP055213003, ISSN: 1022-1336, DOI: 10.1002/marc.201400578
- MOHAMED BOULARAS ET AL: "Supporting informations, Design of Smart Oligo(ethylene glycol)-Based Biocompatible Hybrid Microgels Loaded with Magnetic Nanoparticles", MACROMOLECULAR RAPID COMMUNICATIONS, vol. 36, no. 1, 25 November 2014 (2014-11-25), pages 79 - 83, XP055213003, ISSN: 1022-1336, DOI: 10.1002/marc.201400578
- WEITAI WU ET AL: "Multi-functional core-shell hybrid nanogels for pH-dependent magnetic manipulation, fluorescent pH-sensing, and drug delivery", BIOMATERIALS, ELSEVIER SCIENCE PUBLISHERS BV., BARKING, GB, vol. 32, no. 36, 31 August 2011 (2011-08-31), pages 9876 - 9887, XP028316441, ISSN: 0142-9612, [retrieved on 20110902], DOI: 10.1016/J.BIOMATERIALS.2011.08.082

## Description

L'invention porte sur des microgels de poly(oligo-(éthylène glycol) méthacrylate), sur leur procédé de préparation en milieu aqueux et leurs utilisations dans différents domaines d'applications tels que, la cosmétique, la pharmacie et le diagnostic médical.

Ces microgels présentent l'avantage d'être monodisperses, pH-sensibles et thermosensibles et de pouvoir incorporer des molécules organiques ou des particules inorganiques. Des films préparés à partir de solutions colloïdales de ces microgels non chargés en nanoparticules inorganiques présentent des propriétés optiques et électromécaniques très avantageuses.

### ART ANTERIEUR

Il existe plusieurs chimies de microgels thermosensibles. Les principales sont à base de poly(N-isopropylacrylamide) (PNIPAM), et plus rarement de poly(N-vinylcaprolactame) (PVCL) ou de poly(oligo-(éthylène glycol) méthacrylate).

Les différentes voies de synthèse de microgels poly(oligo-(éthylène glycol) méthacrylate) qui ont été décrites dans la littérature font intervenir des combinaisons de monomères tels le monomère di(éthylène glycol) méthyl éther méthacrylate (M(EO)₂MA, plutôt hydrophobe), et le monomère penta(éthylène glycol) méthyl éther méthacrylate (M(EO)₅MA plutôt hydrophile).

La synthèse d'un microgel thermosensible et pH-sensible, constitué d'un coeur thermosensible à base de P(M(EO)₂MA-*co*-M(EO)₅MA) et d'une écorce à base de mélange de poly(oligo-(éthylène glycol) méthacrylate) et de poly(acide acrylique) (P(M(EO)₂MA-*co*-M(EO)₅MA-co-AA)) a été décrite par Chi, C., T. Cai, and Z. Hu, Oligo(ethyleneglycol)-Based Thermoresponsive Core-Shell Microgels. Langmuir, 2009. 25: p. 3814-3819. Ces microgels ont une structure coeur/écorce avec un coeur hydrophobe et une écorce hydrophile.

Il a été suggéré d'incorporer des molécules biologiquement actives dans des microgels à base de poly(oligo-(éthylène glycol) méthacrylate).

Par exemple un microgel thermosensible constitué d'un coeur poly(M(EO)₂MA) et d'une écorce poly(M(EO)₂MA-co-M(EO)₅MA) et des nanocapsules de poly(M(EO)₂MA-co-OEGMA) obtenues par greffage du polymère sur une particule de silice sacrificielle ont été proposés pour la délivrance de principe actif respectivement par Zhou, et al., Engineering oligo(ethylene glycol)-based thermosensitive microgels for drug delivery applications. Polymer, 2010. 51: p. 3926-3933 ; et par Wang, et al., Préparation of biocompatible nanocapsules with temperature-responsive and bioreducible properties, Journal of Materials Chemistry, 2011. 21: p. 15950.

Les microgels thermosensibles hybrides (ou nanocomposites) connus contenant des nanoparticules inorganiques sont des microgels à base de poly(N-isopropylacrylamide) (PNIPAM). Ces microgels présentent l'inconvénient de ne pas être biocompatibles.

Une première approche pour préparer ces matériaux consiste à synthétiser les microgels en présence des nanoparticules. Cette stratégie rend difficile la fabrication de ces microgels hybrides en raison de la complexité de la polymérisation. Le taux de nanoparticules incorporés est généralement faible et les microgels sont polydisperses. De plus, elle conduit généralement à une architecture de type coeur-écorce au sein de laquelle les nanoparticules sont réparties de manière uniforme.

Une seconde approche consiste à synthétiser, dans un premier temps, les microgels thermosensibles fonctionnalisés par des groupements ioniques. Par la suite, les nanoparticules inorganiques sont incorporées par co-précipitation des sels précurseurs des nanoparticules. La demande WO2004/081072 décrit par exemple des microgels PNIPAM présentant des unités anioniques acrylate de sodium (-COO⁻ Na⁺) et la co-précipitation *in situ* de sels précurseurs de nanoparticules diverses telles que des particules magnétiques (Fe₃O₄), des particules d'or (Au), et des particules Quantum dots (CdTe, CdS).

Une troisième approche consiste à incorporer les nanoparticules inorganiques par transfert de solvant. Des nanoparticules inorganiques hydrophobes sont synthétisées et dispersées dans une phase organique. Les microgels thermosensibles sont ajoutés dans la solution contenant les nanoparticules puis le tout est transféré en solution aqueuse, ce qui encapsule les nanoparticules dans les microgels. Cette méthode a fait l'objet d'un brevet en utilisant des microgels poly(N-isopropylacrylamide) et des nanoparticules de Quantum dots (CdS) pour des applications en photoluminescence (US 7914710).

Une quatrième et dernière approche consiste à synthétiser des microgels portant des charges ioniques et à faire adsorber des nanoparticules de charges opposées en surface du microgel. La préparation de latex poly(styrène-co-N-isopropylacrylamide) présentant des groupements cationiques 2-aminoéthyl méthacrylate (AEMA) a été décrite dans la demande WO 1997/045202. Des nanoparticules magnétiques γ-Fe₃O₃ chargées négativement en surface ont été adsorbées à la surface des latex par interaction électrostatique. Les nanoparticules ont ensuite été piégées dans la structure par synthèse d'une nouvelle écorce externe de PNIPAM à la surface des latex hybrides.

Plus récemment, des nanoparticules portant une charge positive (TiO₂) ont été incorporées dans une structure de microgel PNIPAM chargé par des unités comprenant un groupe acrylate (COO⁻) (US 8158005).

L'utilisation des microgels, notamment des microgels thermosensibles ayant des propriétés magnétiques, requiert dans certains domaines leur préparation sous forme de films minces.

La formation de films minces composés de microgels préalablement dispersés dans une phase aqueuse est un procédé de synthèse difficile car il nécessite de concilier deux facteurs opposés. Les microgels dispersés en phase aqueuse sont stabilisés par des charges répulsives qui empêchent les microgels de s'agréger ou de sédimenter. Or, les microgels doivent interagir les uns aux autres pour former des couches de microgels afin de former un film. Plusieurs procédés ont dû être développés pour les former.

Un premier procédé d'auto-assemblage de microgels sur surface modifiée consiste à ancrer des microgels à la surface d'un substrat préalablement traité pour créer des charges ioniques sur sa surface. Les microgels présentent des groupements ioniques issus généralement de l'amorceur de polymérisation ou d'un co-monomère ionique. Les microgels peuvent être ancrés à la surface d'un substrat par interaction électrostatique avec des groupements de charges opposées. Cette technique permet de déposer une couche mince de microgel sur le substrat (*monolayer technique*)*,* mais il est également possible de multiplier ces couches par des traitements successifs de surface (*Layer-by-Layer technique*). Des microgels à base de poly(N-isopropylacrylamide-co-acide acrylique) ou P(NIPAM-*co*-AA) ont été déposés selon cette technique sur des substrats greffés par des groupements 3-aminopropyltriméthoxysilane (APTMS). Après chaque couche de microgel, un polymère présentant des charges positives (poly(allylamine hydrochloride) PAH ou poly(éthylène imine) PEI) a été ajouté sur le substrat modifié afin de régénérer les charges positives pour un pH inférieur à la constante d'acidité des amines.

Les films de microgels déposés sur substrat ont été utilisés pour la délivrance de principe actif tel que l'insuline pour le traitement du diabète (Nolan, C.M., M.J. Serpe, and L.A. Lyon, Thermally Modulated Insulin Release from Microgel Thin Films. Biomacromolecules, 2004. 5(5): p. 1940-1946) ou la doxorubicine pour le traitement de cellules cancéreuses (Serpe, M.J., et al., Doxorubicin Uptake and Release from Microgel Thin Films. Biomacromolecules, 2005. 6(1): p. 408-413).

Cette technique a l'avantage de contrôler les paramètres inhérents à l'élaboration de film mince de microgels (tels que l'épaisseur de film et la structuration des microgels.). L'inconvénient de ce procédé réside dans sa complexité d'élaboration qui nécessite d'utiliser des microgels particuliers (microgels chargés) et des substrats préalablement traités. Ceci empêche toute utilisation directe des microgels sur une surface quelconque.

Un second procédé d'élaboration d'un film d'hydrogel encapsulant des particules colloïdales consiste à encapsuler les particules dans un hydrogel afin de former un film souple et humide. Ce procédé est étudié principalement pour des applications photoniques. L'idée est d'associer les propriétés optiques de particules assemblées avec les propriétés mécaniques des hydrogels à l'état humide. Quelques exemples sont décrits dans la littérature et utilisent principalement des particules poly(styrène) sous forme de sphères dures. E. Tian et al. ont assemblé des particules de Poly(Styrene-co-Methacrylate de méthyl-co-acide acrylique) en plusieurs couches structurées et ont encapsulé le tout dans un hydrogel de poly(acrylamide) (Tian, E., et al., Colorful humidity sensitive photonic crystal hydrogel. Journal of Materials Chemistry, 2008. 18: p. 1116-1122). L'association des deux entités a permis de développer des hydrogels photoniques. Plus récemment, H. Jiang et al. ont également encapsulé des particules de poly(styrène) dans un hydrogel de poly(alcool vinylique) ou PVA (Jiang, H., et al., Photonic crystal pH and metal cation sensors based on poly(vinyl alcoho/) hydrogel. New Journal of Chemistry, 2012. 36: p. 1051-1056.).

Plus récemment, H. Kim et al. ont assemblé des particules magnétiques d'oxyde de fer (Fe₃O₄) enrobé d'oxyde de silice (SiO₂) sous l'effet d'un champ magnétique et ont encapsulé cet assemblage dans un mélange de monomère poly(éthylène glycol) diacrylate et d'un photo-amorceur. La photopolymérisation du mélange permet de figer les particules dans une résine de poly(éthylène glycol acrylate). Les particules magnétiques figées dans la masse ont des propriétés photoniques pouvant être défini par le champ magnétique appliqué (US 2012/0028834). Cette technique présente l'avantage de produire des films de microgels plus souples car ces derniers sont supportés par un hydrogel « mou » en solution, et non un support solide. Cependant, la réalisation de ces films de particules reste complexe et différentes étapes de polymérisation sont nécessaires ce qui empêche tout auto-assemblage spontané lors d'une utilisation directe des particules.

Un troisième procédé de formation de films de microgels consiste à ajouter des fonctions réactives à la surface des microgels. Cette fonctionnalité est apportée par ajout d'un co-monomère lors de la synthèse des microgels. Ces fonctions réactives peuvent soit former des liaisons covalentes les unes avec les autres, soit former des liaisons covalentes par réaction avec une autre entité. Il est alors possible de former des nœuds de réticulation entre chaque microgels, le tout donnant un film composé de microgels liés chimiquement les uns aux autres. Ce procédé d'auto-assemblage est étudié principalement pour des applications photoniques. Le procédé d'auto-assemblage dépend du type de fonctionnalité ajoutée lors de la synthèse des microgels. Une grande majorité de ces travaux ont porté sur l'assemblage de microgel à base de PNIPAM.

Une première approche consiste à ajouter du poly(acide acrylique) (ou PAA) au sein de microgels de PNIPAM. L'auto-assemblage des microgels se fait grâce à des interactions faibles entre les fonctions acides carboxyliques du PAA. La somme des interactions permet d'auto-assembler les microgels et de gélifier le milieu.

Une seconde approche consistant à créer des liaisons covalentes a également été proposée, soit par ajout d'un réticulant dans la solution de microgels de PNIPAM, soit par polycondensation de microgels PNIPAM-co-NMA tel (NMA : N-methylol acrylamide ou N-hydroxymethyl acrylamide). Les microgels sont auto-assemblés par simple séchage d'une dispersion de microgels. La formation du film nécessite une étape supplémentaire de post-polymérisation thermique du réticulant ou de condensation thermique et acido-basique catalysée du NMA.

Une étude a utilisé des microgels de dérivé oligo-(éthylène glycol) méthacrylate présentant des fonctions polymérisables en surface. Ces microgels ont été réticulés par photo-polymérisation UV lors de leur assemblage pour des applications photoniques (US 2010/0076105). Cependant, la filmification requiert un temps de séchage très long d'environ quelques semaines.

L'objectif de l'invention est d'utiliser un microgel oligo-(éthylène glycol) méthacrylate qui présente au moins un des avantages suivants par rapport aux microgels de l'art antérieur : monodisperse ; pH-sensible ; biocompatible ; capable de former des microgels hybrides (ou nanocomposites) par adsorption de nanoparticules de charges opposées; capable de s'auto-assembler en plusieurs couches par simple procédé de séchage ; capable de former un film transparent ; capable de former un film cohésif et élastique ; sous forme d'un film, capable de générer un potentiel électrique par effet de compression ; sous forme d'un film, capable de diffracter la lumière générant ainsi une couleur.

Il a été découvert dans le cadre de la présente invention que certains poly(oligo-(éthylène glycol) méthacrylate) ont un fort potentiel filmogène par évaporation de l'eau à température ambiante.

Les films de microgels présentent l'avantage d'être totalement auto-supportés. Les microgels n'étant pas encapsulés ni supportés, lors de la filmification l'interaction entre les microgels et le substrat sur lesquels ils sont déposés, par exemple la peau, est maximale. Les films sont obtenus par simple séchage à température ambiante et aucun amorceur radicalaire n'est nécessaire. Les microgels contenant ou non des nanoparticules sont capables de s'auto-assembler en plusieurs couches pour former un film transparent.

Il a également été découvert dans le cadre de la présente invention que les microgels poly(oligo-(éthylène glycol) méthacrylate) sont des matériaux polyélectrolytes capables de générer un champ électrique par action mécanique.

### DESCRIPTION DE L'INVENTION

L'invention porte sur un produit cosmétique ou pharmaceutique contenant des microgels poly(oligo-(éthylène glycol) méthacrylate) dotés de propriétés colloïdales ainsi que d'une sensibilité aux variations de température et/ou de pH dans l'eau, grâce à la présence de groupements -COOH éventuellement salifiés.

On entend par « microgel » au sens de l'invention, un polymère réticulé sous forme d'une particule sphérique de taille variant de 100 nm à 500 nm à l'état sec (c'est-à-dire contenant moins de 2% en poids d'eau), de préférence entre 350 et 450 nm, de préférence encore de l'ordre de 400 nm. Le microgel de l'invention est un microhydrogel en ce sens qu'il est susceptible d'être obtenu par un procédé de copolymérisation en phase aqueuse de plusieurs monomères. Le microgel de l'invention n'a pas une structure coeur/écorce : les monomères qui le composent sont répartis de façon uniforme sur l'ensemble du volume de la particule, ce qui lui confère des propriétés particulières.

Les microgels peuvent présenter l'avantage d'être à la fois monodisperses, thermosensibles, pH-sensibles et biocompatibles. Les microgels de l'invention peuvent être à la fois thermosensibles et biocompatibles, contrairement aux microgels thermosensibles de l'art antérieur qui sont généralement des structures à base de poly(alkylacrylamide).

Les microgels présentent l'originalité de comprendre un mélange d'unités répétitives d'oxyde d'éthylène branchés et d'unités comprenant un groupement acide carboxylique (-COOH) ou carboxylate (-COO⁻) dont on peut faire varier la teneur en fonction des applications visées. Ces groupements confèrent aux microgels la propriété de pH-sensibilité.

L'invention a ainsi pour objet un produit cosmétique ou pharmaceutique contenant des microgels et au moins un composé choisi dans le groupe constitué par les agents tensio-actifs, les huiles, les molécules produits biologiquement activfes, les pigments et les colorants, les microgels étant sous forme de particules sphériques susceptibles d'être obtenues par un procédé de copolymérisation en phase aqueuse des plusieurs trois monomères suivants répartis de façon uniforme sur l'ensemble du volume des particules dont ::
- le di(éthylène glycol) méthyl éther méthacrylate (M(EO)2MA),
- un oligo(éthylène glycol) méthyl éther méthacrylate (M(EO)nMA) n étant un entier allant 3 à 12,
- un monomère de formule CR1R2=CR3R4 dans laquelle R1, R2, R3 et R4 représentent un hydrogène, un halogène ou un groupement hydrocarboné, à la condition qu'au moins un des quatre groupements comprenne un groupement -COOH ou -COO-M+, tel que M+ représente un cation,

en présence d'un agent réticulant,
les microgels ne comprenant pas des nanoparticules inorganiques.

Dans la suite du texte, on désigne sous le terme « -COOH », la forme acide - COOH ou salifiée -COO-M+, pour alléger l'écriture.

M(EO)₂MA représente par exemple 50 à 90% en moles du nombre total de moles des monomères, M(EO)ₙMA représente de préférence 10 à 50% en moles du nombre total de moles des monomères, le monomère de formule CR₁R₂=CR₃R₄ représente de préférence 0,1 à 20 % en moles du nombre total de moles des monomères, la somme de ces trois teneurs étant égale à 100%.

La ratio molaire entre M(EO)₂MA et M(EO)ₙMA est de préférence compris entre 1 :1 et 20 :1, par exemple entre 5 :1 à 10 :1. L'expression « compris entre » au sens de l'invention exclut les bornes numériques qui lui succèdent. En revanche, l'expression « allant de ...à » vise à inclure les bornes exprimées.

Le nombre de moles de monomère de formule CR₁R₂=CR₃R₄ peut être compris entre 0 et 20 mol%, par exemple aller de 0,1 à 5 mol % du nombre total de moles totale des trois monomères.

Selon un mode de réalisation, M(EO)₂MA représente par exemple 80 à 90% en moles du nombre total de moles des trois monomères, M(EO)ₙMA représente de préférence 5 à 15% en moles du nombre total de moles des monomères et l'acide méthacrylique représente de préférence 0,1 à 10 % en moles du nombre total de moles des monomères, la somme de ces trois teneurs étant égale à 100%.

Le monomère de formule CR₁R₂=CR₃R₄ est de préférence tel que R₁ et R₂, représentent chacun un hydrogène, R₃ représente H ou un groupement alkyle, de préférence en C1-C6, éventuellement substitué par -OH ou -COOH, et R₄ représente indépendamment de R₃ le groupement-COOH ou un groupement alkyle, de préférence en C1-C6, éventuellement substitué à -OH ou -COOH. Le groupement alkyle peut être méthyle, éthyle ou n-butyle. Selon un mode de mise en œuvre particulier, R₁ et R₂, représentent chacun un hydrogène et R₃ et R₄ représentent indépendamment -H, - COOH, ou -CH₂-COOH.

Le monomère de formule CR₁R₂=CR₃R₄ peut être par exemple choisi parmi les acides méthyl acrylique, méthyl méthacrylique, éthyl acrylique, éthyl méthacrylique, n-butyl acrylique, n-butyl méthacrylique.

Selon un mode de réalisation, le monomère de formule CR₁R₂=CR₃R₄ peut être l'acide méthacrylique ou l'acide itaconique. L'acide acrylique peut être exclu de la définition du monomère de formule CR₁R₂=CR₃R₄ dans certains cas.

L'agent réticulant peut être choisi dans le groupe constitué par les oligo(éthylène glycol) diacrylate comprenant de 1 à 10 motifs éthylène glycol, 1,3-Butanediol diacrylate, 1,4-Butanediol diacrylate, 1,6-Hexanediol diacrylate, Pentaerythritol diacrylate monostearate, Glycerol 1,3-diglycerolate diacrylate, Neopentyl glycol diacrylate, Poly(propylene glycol) diacrylate, 1,6-Hexanediol éthoxylate diacrylate, Trimethylolpropane benzoate diacrylate, éthylène glycol diméthacrylate, 1,3-Butanediol diméthacrylate, 1,4-Butanediol diméthacrylate, 1,6-Hexanediol dimethacrylate, glycerol dimethacrylate, N,N divinylbenzene, N,N-Methylenebisacrylamide, N,N-(1,2-Dihydroxyethylene)bisacrylamide, Poly(ethylene glycol) diacrylamide, Allyl disulfide, Bis(2-methacryloyl)oxyethyl disulfide et N,N-Bis(acryloyl)cystamine.

L'agent réticulant représente par exemple de 1 à 5 % en moles du nombre total de moles des trois monomères.

Les monomères utilisés sont de préférence le di(ethylene glycol) methyl ether méthacrylate (M(EO)₂MA, Mn 250 g.mol⁻¹), l'oligo(ethylene glycol) methyl ether méthacrylate (M(EO)₉MA, Mn 475 g.mol⁻¹), l'acide méthacrylique (MAA).

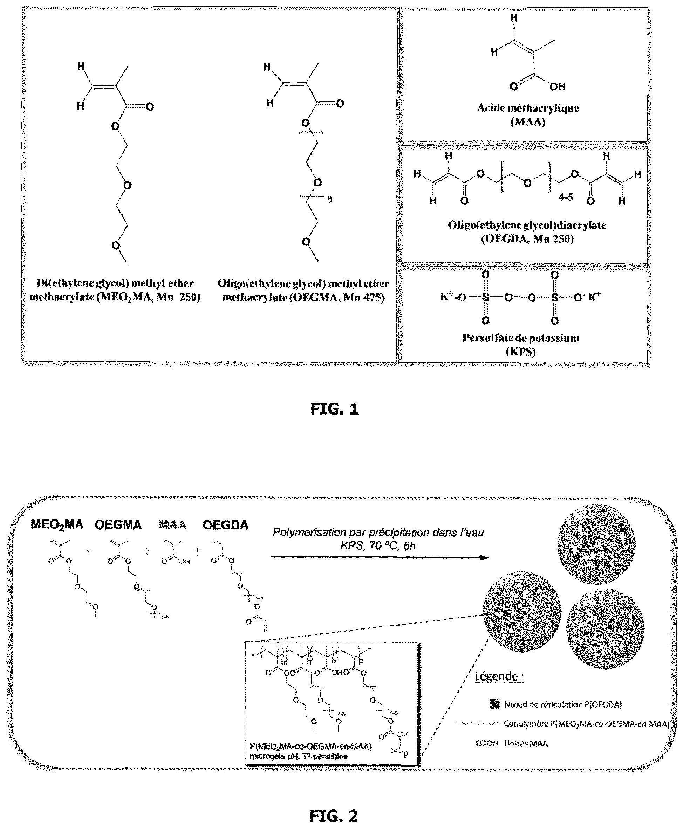

L'agent réticulant est par exemple l'oligo(ethylene glycol)diacrylate comprenant 4 à 5 unités oxyde d'éthylène (OEGDA, Mn 250 g.mol⁻¹). Les structures chimiques des monomères et de l'agent réticulant préférés sont représentées sur La Figure 1.

La taille moyenne d'un microgel peut varier selon qu'il est sec ou en solution aqueuse : ainsi un microgel à l'état sec peut atteindre quatre fois sa taille initiale lorsqu'il est placé en solution aqueuse à 20°C. La taille moyenne d'un microgel de l'invention à l'état sec peut aller de 100 à 1000 nm. La fonction de distribution radiale hydrodynamiques des microgels mesurée à un angle de 60° et à une température de 20°C, est avantageusement inférieure à 1,1, ce qui confère aux microgels la qualité de monodisperses.

Les microgels peuvent comprendre des particules organiques ou inorganiques : on les nomme couramment dans ce cas des microgels hybrides. Les particules introduites ont de préférence une taille comprise entre 1 et 150 nm, par exemple entre 5 et 50 nm, et sont nommées des nanoparticules..

Certains microgels hybrides monodisperses, thermosensibles et magnétiques à base de poly(oligo-(éthylène glycol) méthacrylate) sont obtenus à partir d'au moins deux monomères
- le di(éthylène glycol) méthyl éther méthacrylate (M(EO)₂MA), et
- un oligo(éthylène glycol) méthyl éther méthacrylate (M(EO)ₙMA) n étant un entier allant 3 à 12, et éventuellement en présence d'un troisième monomère :
- un monomère de formule CR₁R₂=CR₃R₄ dans laquelle R₁, R₂, R₃ et R₄ représentent un hydrogène, un halogène ou un groupement hydrocarboné, à la condition qu'au moins un des quatre groupements comprenne un groupement -COOH ou -COO⁻M⁺, tel que M⁺ représente un cation.

Un procédé de préparation des microgels hybrides monodisperses, thermosensibles et magnétiques à base de poly(oligo-(éthylène glycol) méthacrylate) consiste à :
- préparer une dispersion colloïdale de nanoparticules chargées positivement en surface et placées en solution aqueuse,
- préparer une dispersion colloïdale aqueuse de microgels tels que décrits précédemment,
- mélanger les deux dispersions colloïdales et ajuster du pH au-dessus du point isoélectrique des nanoparticules.

Selon un mode de réalisation, les nanoparticules sont chargées positivement à leur surface lorsqu'elles sont placées en solution aqueuse si bien que les microgels hydrides peuvent être préparés par simple mélange de deux dispersions colloïdales : une première dispersion colloïdale de microgels et une deuxième dispersion colloïdale de nanoparticules. Les paramètres clés permettant la réussite de ce procédé résident, d'une part, dans l'ajout de groupements carboxyliques ou carboxylates répartis de manière homogène au sein du microgel et, d'autre part, dans la charge positive de surface des nanoparticules. Le tout permet d'encapsuler de manière contrôlée les nanoparticules au sein du microgel, tout en conservant les propriétés colloïdales et thermosensibles du matériau final. L'architecture hybride des microgels ainsi que leurs propriétés thermosensibles sont démontrées dans cette invention. L'incorporation des nanoparticules au sein des microgels est tout d'abord démontrée avec un taux important et quantitatif de nanoparticules magnétiques encapsulés (taux de charge testés allant de 0 à 33 % massique de nanoparticules par microgel hybride). Les propriétés thermosensibles en solution aqueuse des microgels hybrides sont également démontrées quelques soit le taux de charge en nanoparticules magnétiques.

Selon un mode de réalisation, les nanoparticules sont des pigments, des colorants ou des filtres solaires couramment utilisés dans le domaine de la cosmétique, de l'agroalimentaire ou de la pharmacie.

La préparation de microgels hybrides thermosensibles à base de poly(oligo-(éthylène glycol) méthacrylate) et de nanoparticules peut être réalisée préférence par un procédé de mélange des deux constituants sous forme de dispersions colloïdales. Les nanoparticules placées en solution aqueuse sont susceptibles d'être chargées positivement en surface lorsqu'elles sont mises en contact avec les microgels. La stabilité des microgels hybrides est obtenue par une augmentation de pH au-dessus du point isoélectrique des nanoparticules inorganiques tout en préservant l'encapsulation des nanoparticules.

Ce procédé présente l'avantage d'être simple de mise en oeuvre. La répartition homogène des groupements acides carboxyliques ou carboxylate au sein du microgel et, le choix de nanoparticules chargées positivement en surface permet d'encapsuler de manière contrôlée les nanoparticules au sein du microgel, tout en conservant les propriétés colloïdales et thermosensibles du matériau final.

Les microgels hybrides de l'invention peuvent contenir jusqu'à 50% en poids, notamment jusqu'à 35% en poids de nanoparticules sans perdre leurs propriétés colloïdales, pH et thermosensibles. Le taux de nanoparticules par microgels hybrides peut être déterminé par analyse thermogravimétrique (TGA).

Les microgels décrits précédemment comprenant éventuellement des nanoparticules sont capables de s'auto-assembler pour former un film constitué d'une ou de plusieurs couches de microgels, par un procédé de séchage ou d'évaporation d'une suspension aqueuse desdits microgels.

Les films formés sont cohésifs et élastiques. Les microgels peuvent ainsi être utilisés comme agent filmogène dans des compositions cosmétiques, de manière à améliorer la tenue de ces compositions sur les matières kératiniques. Après séchage, les films ne se re-dispersent pas lorsqu'ils sont immergés dans l'eau

Les microgels et les films qu'ils forment peuvent générer un potentiel électrique par effet de compression (effet Donan). Les films sont préparés à partir de microgels comportant des sites ioniques issus des fonctions carboxylate (COO⁻). Ces sites ioniques sont contraints dans la structure et créent des polarisations au sein du microgel (e.g. microgels polyélectrolytes). Lorsque le film est soumis à une pression, un mouvement des contre-ions contribue à créer une polarisation au sein même du film ce qui génère une différence de potentiel électrique entre la surface et la masse du film. Les inventeurs ont trouvé que la présence d'acide méthacrylique améliore les propriétés mécano-électriques des films.

L'auto-assemblage des microgels monodisperses permet également une diffraction de la lumière générant ainsi une couleur. Ces propriétés photoniques peuvent être modulées par la composition même des microgels.

Les microgels monodisperses peuvent s'auto-assembler de manière périodique sous forme de cristaux colloïdaux. Cet auto-assemblage particulier permet la diffraction d'une lumière incidente et génère ainsi une couleur observable en fonction de l'angle d'observation. Cet effet varie en fonction de la composition des microgels : **a)** le séchage d'une dispersion de microgels sans nanoparticules induit la formation d'un film sec totalement transparent, incolore et ne diffractant pas la lumière. Alors que ce même film diffracte la lumière à l'état humide avec des couleurs observables en solution. **b)** Le séchage d'une dispersion de microgels contenant des nanoparticules magnétiques (microgels hybrides) induit la formation d'un film sec transparent, coloré (marron) et diffractant la lumière. Dès lors, le film est marron à un angle d'observation de 90°C (couleur issu des nanoparticules magnétiques) et change de couleur en réflexion à des angles d'observation plus faibles.

L'ensemble de ces propriétés permet d'envisager l'utilisation des microgels de l'invention et des films qu'ils forment pour la préparation de produits cosmétiques ou pharmaceutiques. Ces produits peuvent stimuler la peau en générant un courant électrique et éventuellement délivrer une molécule biologiquement active par effet de compression. La molécule biologiquement active peut être encapsulée dans les microgels ou être présente dans le produit

L'invention a donc pour objet un produit cosmétique ou pharmaceutique constitué ou contenant des microgels tels que décrits précédemment et au moins un composé choisi dans le groupe constitué par les agents tensio-actifs, les huiles, les produits biologiquement actifs, les pigments et les colorants.

Le microgel, bien que dépourvu de nanoparticules inorganiquespeut contenir toutes sortes d'ingrédients ou d'excipients utilisés dans le domaine cosmétique et pharmaceutique, de préférence des substances biologiquement actives.

Les microgels peuvent être synthétisés par un procédé de polymérisation par précipitation, à partir des monomères préalablement solubilisés en solution aqueuse.

La présente demande décrit un procédé de polymérisation par précipitation d'un microgel de poly(éthylène glycol-méthacrylate) tel que décrit précédemment, comprenant une étape de mise en contact en phase aqueuse en présence d'un agent réticulant des trois monomères décrits précédemment, à une température comprise entre 40 et 90°C. Le procédé de l'invention ne requiert pas la présence d'un agent tensio-actif tel que le SDS (dodecyl sulfate sodium).

La polymérisation des monomères peut être amorcée par ajout d'un amorceur radicalaire hydrosoluble, par exemple le persulfate de potassium (KPS) à une température comprise entre 40 et 90°C, de préférence de l'ordre de 70°C.

On préfère ajouter une solution aqueuse du monomère de formule CR₁R₂=CR₃R₄ de façon graduelle dans une solution aqueuse des deux autres monomères, de manière à garantir la répartition homogène des groupements -COOH dans les microgels qui précipitent.

A la température de polymérisation, le polymère formé est hydrophobe et précipite dans le milieu réactionnel aqueux sous forme de particules sphériques, la présence du réticulant OEGDA au cours de la polymérisation permet de figer les particules sous cette forme sphérique en créant des noeuds de réticulation.

Les microgels présentent l'avantage d'être de structure homogène, car les groupements -COOH et les noeuds de réticulation sont répartis de façon régulière sur tout leur volume.

Les films de microgels sont avantageusement préparés par un procédé de séchage ou d'évaporation de solvant à 20°C, par exemple en partant d'une dispersion colloïdale de microgels monodisperses à une concentration massique pouvant varier de 1.4 à 5 wt% dans l'eau.

Selon ce procédé, au moins un premier volume de solution peut être laissé sécher jusqu'à complète évaporation de l'eau à température ambiante. Cette étape peut être répétée plusieurs fois pour obtenir un film composé de plusieurs couches de microgels monodisperses et d'épaisseur pouvant varier entre 350 et 450 micromètres à l'état sec.

Le produit cosmétique ou les microgels peuvent être utilisés dans un procédé de soin ou de maquillage cosmétique qui consiste à appliquer sur la peau les microgels ou le produit cosmétique décrit précédemment. Les microgels peuvent être utilisés dans différents domaines d'applications tels que la cosmétique, la pharmacie et le diagnostic médical.

### BREVE DESCRIPTION DES FIGURES

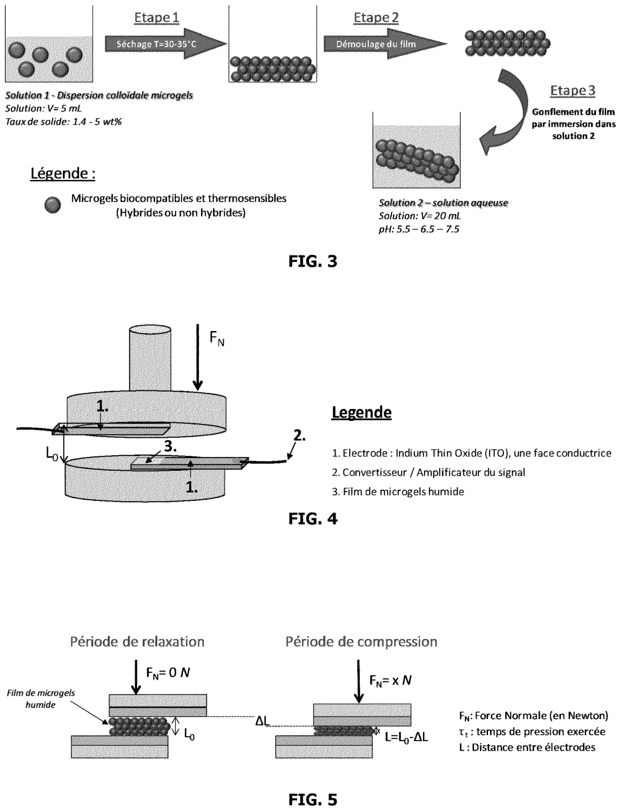

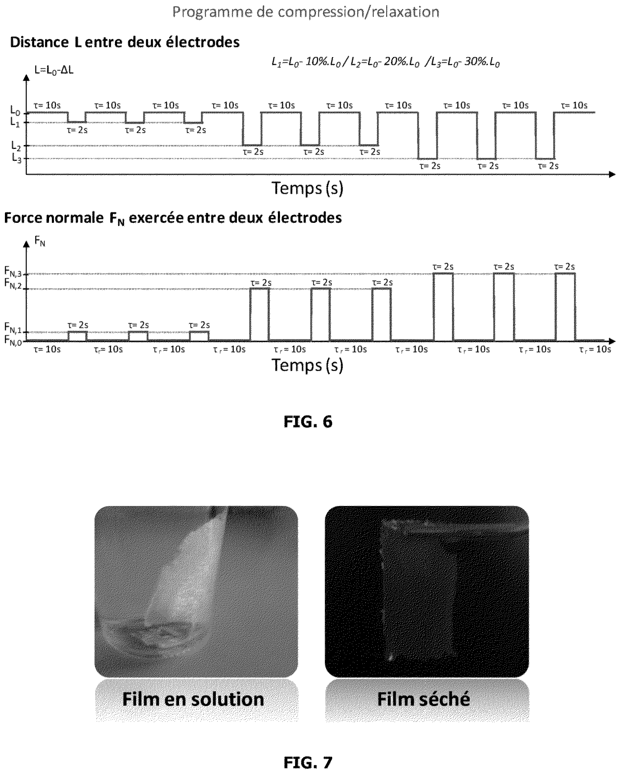

**La** **Figure 1** présente les structures chimiques des monomères utilisés pour la synthèse des microgels biocompatibles de l'invention.
**La** **Figure 2** est un schéma de synthèse des microgels biocompatibles pH-sensibles et thermo-sensibles à base de poly(MEO₂MA-*co*-OEGMA-*co*-MAA) de l'invention.
**La** **Figure 3** représente un schéma du procédé de formation des films de microgels selon l'invention.
**La** **Figure 4** représente un montage de caractérisation de l'effet mécano-électrique des films de microgels de l'invention.
**La** **Figure 5** est une représentation schématique de la période de compression et de relaxation d'un film de microgels de l'invention.
**La** **Figure 6** représente le schéma d'un programme de compression et de relaxation de microgels de l'invention.
**La** **Figure 7** est un cliché de films de microgels de l'invention à l'état sec et gonflé en solution.

L'invention est également illustrée par les exemples suivants.

### EXEMPLE 1 : Synthèse d'un microgel à base de poly(oligo-(éthylène alycol) méthacrylate) selon l'invention

On a utilisé les monomères suivants : di(éthylène glycol) méthyl éther méthacrylate (M(EO)₂MA, Mn 250 g.mol⁻¹), oligo(éthylène glycol) méthyl éther méthacrylate (M(EO)₄₋₅MA encore noté OEGMA dans la suite, Mn 475 g.mol⁻¹), et acide méthacrylique (MAA). L'agent de réticulation était l'oligo(éthylène glycol)diacrylate (OEGDA, Mn 250 g.mol⁻¹).

### Protocole expérimental:

0.966 g de MEO₂MA (5.14×10⁻³ mol), 0.272 g d'OEGMA (5.73×10⁻⁴ mol) et 0.029 g de OEGDA (1.17×10⁻⁴ mol) sont introduits dans volume de 57.5 mL d'eau et laissés sous agitation magnétique jusqu'à complète dissolution des monomères. Le mélange est alors filtré et introduit dans un tricol d'un volume de 250 mL équipé d'un agitateur mécanique avant d'être dégazés à l'azote durant 45 min sous agitation mécanique (150 rpm). Une solution aqueuse de MAA (0.026 g, 3.05×10⁻⁴ mol dissous dans 2 mL d'eau) est alors introduite dans le milieu réactionnel. Le mélange est laissé à 70°C durant 20 min avant d'introduire une solution aqueuse de persulfate de potassium (KPS, 0.0143 g dissout dans 2.5 mL d'eau) préalablement dégazé à l'azote. L'ajout de KPS permet d'amorcer la polymérisation et le milieu réactionnel est laissé sous agitation mécanique (50 rpm) à 70°C durant 6h.

La polymérisation est ensuite arrêtée par ajout d'oxygène et laissée refroidir jusqu'à température ambiante. Les microgels sont alors séparés du milieu réactionnel par centrifugation (10 000 rpm, 30 min) et le milieu réactionnel est remplacé par de l'eau pure (de grade milliQ), l'étape est répétée cinq fois.

La solution finale se compose alors d'une dispersion colloïdale de microgels P(MEO₂MA-*co*-OEGMA-*co*-MAA) en phase aqueuse, cette dispersion est gardée à température ambiante.

### Propriété du microgel

La synthèse des microgels a été caractérisée par suivi cinétique des monomères en utilisant la spectroscopie à résonance magnétique nucléaire du proton (RMN ¹H). On observe une conversion totale des monomères ainsi qu'une composition homogène des microgels avec une distribution homogène des nœuds de réticulation et des unités acide méthacrylique.

Le rendement final en microgel réticulé a été analysé par extrait sec du milieu réactionnel et permet de déterminer un rendement de 70 % massique en microgel réticulé.

Le taux d'acide méthacrylique incorporé a été déterminé par dosage acido-basique des microgels purifiés. Le caractère pH-sensible des microgels en solution aqueuse ainsi que l'incorporation de 70% molaire du monomère MAA initial a pu être vérifiée.

Les microgels ont été observés par microscopie électronique à transmission (MET) et leurs tailles déterminées par diffusion dynamique de la lumière. Les microgels observés sont monodisperses, avec une taille de 400 nm à l'état séché et pouvant aller jusqu'à 1000 nm à l'état humide.

### EXEMPLE 2: Préparation de solutions de microael à base de poly(oliao-(éthylène alycol) méthacrylate)

1.2 mL d'une solution de microgels préparés selon l'Exemple 1 (à 15 g.L⁻¹ de microgels) est dispersé dans 10 mL d'une solution d'eau pure (grade milliQ). Le pH de la dispersion est ajusté par ajout d'une solution d'acide chlorhydrique ou d'hydroxyde de potassium à 0.1 mol.L⁻¹. Le mélange est laissé sous agitation magnétique jusqu'à stabilisation du pH. La taille des microgels en solution est mesurée par diffusion dynamique de la lumière et la température de solution est contrôlée au cours de l'analyse.

En étudiant la taille des microgels en solution par diffusion dynamique de la lumière, il a été possible d'évaluer l'impact du pH et de la température du milieu sur la capacité des microgels à gonfler ou à se contracter dans l'eau.

Les microgels sont sensibles aux variations de pH du milieu passant d'une taille de 400 nm à pH < 5.5 à une taille de 1000 nm à pH > 6.0.

Les microgels sont thermosensibles et passent d'un état gonflé à 20°C à un état contracté à température supérieure. La température de contraction est fonction du pH (35°C à pH < 6.0 et 55°C à pH > 7.0). Enfin, le volume du microgel gonflé à 20°C diminue jusqu'à 3 fois par rapport à son volume initial lorsque la température passe au-delà de la température de contraction.

### EXEMPLE 3 : Films de microaels

On a utilisé les compositions de microgels résumées dans le Tableau 1 pour préparer des films.

**Tableau 1. Composition en monomères des microgels**

| | **Microgels P(MEO₂MA-co-OEGMA-co-MAA)** | |
|---|---|---|
| Microgel | **1** | **2** |
| **MEO₂MA** (mmol/L) | 88.4 | 83.9 |
| **OEGMA** (mmol/L) | 9.82 | 9.36 |
| **MAA** (mmol/L) | 0 **^{a}** | 4.99 **^{b}** |
| **OEGDA** (mmol/L) | 1.92 | 1.92 |
| **KPS** (mmol/L) | 0.86 | 0.86 |
| **Morphologie des microgels** | 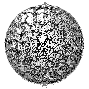 | 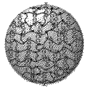 |

| | | |
|---|---|---|
| **^{a} Microgel 1** contient 0 mol% d'unités MAA ; **^{b} Microgel 2** contient 3.5 mol% d'unités MAA incorporés dans le microgel (soit 70 % de monomère MAA initial) ; | | |

Les films sont préparés par un procédé de séchage présenté sur la Figure 3. Partant d'une dispersion colloïdale de microgels monodisperses (de taille pouvant varier entre 500 et 1000 µm en solution) à une concentration massique en microgels variant de 1.4 à 5 wt% dans l'eau **(solution 1).** Un volume constant de solution est introduit dans un moule en plastique et laissé sécher jusqu'à complète évaporation de l'eau (Etape 1 de la Figure 3). Le film restant au fond du moule est alors composé de plusieurs couches de microgels monodisperses et totalement sec (de taille pouvant varier entre 350 et 450 µm à l'état sec). Le film est soigneusement récupéré et réintroduit dans une solution aqueuse **(solution 2).** On fait varier différents paramètres: **1.)** La concentration massique en dispersion de 1.4 à 5 wt% permet de faire varier l'épaisseur de film gonflé (fin de l'étape 3 : épaisseur de 200 µm à 1000 µm). **2.)** le pH de **solution 2** est varié entre 5.5 et 7.5.

### Protocole expérimental: Formation des films de microgels à base de poly(oligo-(éthylène glycol) méthacrylate) thermosensibles.

Un volume de 5 mL d'une dispersion colloïdale de microgels à une concentration massique de 1.4 à 5 wt% est introduit dans un moule en plastique et laissé séché à une température de 32°C (+/- 2°C). Après complète évaporation du solvant, le film est soigneusement récupéré puis introduit dans une solution aqueuse et laissé gonfler à température ambiante.

Les films récupérés ont été observés par microscopie à force atomique à l'état sec et caractérisés au rhéomètre à l'état humide (Fin d'**étape 3 de la** **Figure 3**). Les microgels forment un film élastique composés de plusieurs couches de microgels (à **l'étape 2)** et ce quelle que soit la composition des microgels (**microgels 1** à **2** en Tableau 1). A l'inverse, les microgels perdent leurs propriétés mécaniques lorsqu'ils sont gonflés dans l'eau mais ne se re-dispersent pas en solution.

**Microgels 1** et **2** : on a fait varier l'épaisseur de film de 200 à 1000 µm, la multiplication des couches de microgels ne modifie pas le phénomène de « filmification » et les films gardent leur élasticité à l'état sec.

### Evaluation des propriétés mécano-électriques des films

### 1. Caractérisation des propriétés mécano-électriques des films

Les propriétés mécano-électriques des films de microgels ont été étudiées. Il s'agit là de démontrer la capacité des microgels à générer un courant électrique lorsque l'on exerce une pression sur ces microgels. Plus particulièrement, l'idée de l'invention est de générer un courant électrique en pressant le matériau à la surface d'un substrat. Ce potentiel électrique peut être généré à partir d'un matériau présentant des fonctions ioniques attachées de manière covalente (ou matériau polyélectrolyte). En effet, les groupements ioniques étant attachés dans le microgel, seuls les contre-ions de chaque groupement carboxylates ont une mobilité dans le microgel. Lorsqu'une pression/déformation unidirectionnelle est exercée sur ces microgels polyélectrolytes, la mobilité des contre-ions est favorisée créant ainsi une polarisation entre les charges positives des contre-ions mobiles et les charges négatives des groupements carboxylates attachés. Ce gradient ionique se traduit par un potentiel électrique à l'interface. Ainsi, la présence de fonctions ionisables dans le microgel permettrait de créer une polarisation au sein du matériau et de générer un potentiel électrique.

Un montage est utilisé afin de mettre en évidence les propriétés mécano-électriques des films. Pour cela, un rhéomètre Anton Paar MCR301 est utilisé en géométrie plan-plan au sein duquel deux électrodes plates et conductrices à base d'Indium Tin Oxide ou ITO (**entité 1.** de la Figure 4) sont accrochées de part et d'autre de la géométrie. L'électrode inférieure est fixe et l'électrode supérieure est amovible. Un film humide de microgels (**entité 3.** de la Figure 4) est déposé sur la surface de l'électrode inférieure et l'électrode supérieure est abaissée afin d'exercer une compression de force F_{N} sur le film. En contrôlant la distance entre les deux électrodes, il est possible de contrôler la force d'écrasement (F_{N}) exercée sur le film.

Un programme de compression/relaxation est effectué afin de faire varier la force exercée sur le film. Dans un premier temps, l'épaisseur initiale de film humide (notée L₀) a été déterminée et la distance entre les deux électrodes a été progressivement diminuée d'une distance ΔL en abaissant l'électrode supérieure. Le programme se distingue par une période courte d'écrasement (τ= 2 secondes) avec une distance finale L puis une période longue de relaxation (τ= 20 secondes) avec un retour à l'état initial L₀, le tout permet de simuler une action de type « touch-sensitive » sur le film (Figure 5).

A la période d'écrasement, une force normale F_{N} (en newton) est enregistrée. Cette force F_{N} est proportionnelle à l'épaisseur d'écrasement (ΔL). Le programme se compose comme tel : le film est compressé à jusqu'à une distance L= L₀-ΔL avec ΔL= (3x) 10%.L₀, puis (3x) 20%.L₀, puis (3x) 25%.L₀ etc. Un exemple de programme utilisé est donné en Figure 6.

Enfin, les électrodes supérieures et inférieures sont reliées à un convertisseur/amplificateur, afin d'enregistrer la différence de potentiel (notés E) générée entre les deux électrodes tout au long du programme.

### 2. Etude des Films de microgels

### Films de microaels P(MEO₂MA-co-OEGMA-co-MAA)

Les films de microgels biocompatibles P(MEO₂MA-co-OEGMA-co-MAA) ont été caractérisés en faisant varier 3 paramètres :
1.) L'effet de la multiplication des compressions : pour chaque films, une compression de même force est répétée successivement (3 fois comme représenté en Figure 6) et le potentiel de chaque compression est analysé.
2.) Epaisseur de films : deux épaisseurs de films ont été testées afin de déterminer l'impact de l'épaisseur sur la capacité des films à générer un potentiel électrique à l'interface (-200 µm et -900-1000 µm).
3.) Composition en fonctions acide carboxyliques : la composition en unité MAA a été variée de 0 à 3.5 mol% en MAA **(microgels 1** et **2)** afin d'évaluer l'impact des unités MAA sur le potentiel électrique.
4.) Le pH de solution dans lequel les films sont gonflés : le pH permet de varier la quantité de fonction ioniques (COO⁻) au sein du microgel. En effet les fonctions acides carboxyliques sont présentes sous forme de deux espèces protonée (COOH) et déprotonée ou ionisé (COO⁻). La proportion de ces deux espèces dépend du pH de la solution avec une augmentation de l'espèce ionisée COO⁻ lorsque l'on augmente le pH (pH 5.5 →%COO⁻= 0 ; pH 6.5 →%COO⁻= 50 % ; pH 7.5 →%COO⁻= 75%).

**Tableau 2. Récapitulatif des échantillons étudiés.**

| **Effet de l'épaisseur de film** | | | | | |
|---|---|---|---|---|---|
| **Echantillon** | **MAA (mol%)** | **Epaisseur de film gonflé (µm)** | **pH** | **Eₘₐₓ (mV)** | **F_{N} (N)** |
| **Microgel 2** | 3.5 | 180 | 6.5 | 5.4 - 11.1 | 0.38 - 0.40 |
| **Microgel 2** | 3.5 | 850 | 6.5 | 2.0 - 5.6 | 0.30 - 0.35 |

| **Effet de la composition en MAA** | | | | | |
|---|---|---|---|---|---|
| **Echantillon** | **MAA (mol%)** | **Epaisseur de film gonflé (µm)** | **pH** | **Eₘₐₓ (mV)** | **F_{N} (N)** |
| **Microgel 1** | **0** | **275** | 6.5 | 0.43 - 0.9 | 0.30 - 0.35 |
| **Microgel 2** | **3.5** | **180** | 6.5 | 5.4 - 11.1 | 0.38 - 0.40 |

| **Effet du pH** | | | | | |
|---|---|---|---|---|---|
| **Echantillon** | **MAA (mol%)** | **Epaisseur de film gonflé (µm)** | **pH** | **Eₘₐₓ (mV)** | **F_{N} (N)** |
| **Microgel 2** | 3.5 | 310 | **5.5** | 2.0 - 9.9 | 0.7 - 0.9 |
| **Microgel 2** | 3.5 | 180 | **6.5** | 5.4 - 11.1 | 0.38 - 0.40 |
| **Microgel 2** | 3.5 | 350 | **7.5** | 5.5 - 11.2 | 0.18 - 0.23 |
| | | | | | |

### Résultats :

En observant l'évolution du signal électrique au cours de la compression des films de microgels, un effet mécano-électrique est mis en évidence sur l'ensemble des films caractérisées. Cet effet mécano-électrique est fonction de la force F_{N} exercée sur les films avec un potentiel électrique qui augmente avec la force de compression. De plus, une tendance semble se dégager en fonction des paramètres d'analyse :
- Effet de la répétition des compressions : le potentiel électrique enregistré est très important lors de la première compression. Alors que lors de la 2^{ème} et 3^{ème} compression, le potentiel généré est plus faible. Cette première constatation peut être due à un mouvement important des ions à la première compression du film créant un potentiel électrique instantané important (- 12 mV). Après un temps de relaxation de 20 s, les compressions suivantes de même force ne semblent pas suffisantes pour provoquer ce même mouvement des ions avec un potentiel généré qui diminue.
- Effet de l'épaisseur de film : le potentiel électrique généré par la compression des films d'épaisseurs différentes montre un faible effet mécano-électrique lorsque le film est trop épais (E = 2 - 5.6 mV pour F_{N,max} = 0.35 N). A l'inverse, un effet mécano-électrique plus important est visible lorsque l'épaisseur de film est faible allant de 11 à 5 mV pour des forces F_{N} de à 0,38 0,4 N. Une épaisseur trop importante ne permettrait donc pas d'impacter suffisamment sur la mobilité des ions. (Cf. Tableau 2. *Effet de l'épaisseur de film)*
- Composition en fonctions acides carboxyliques : la présence d'acide méthacrylique semble améliorer les propriétés mécano-électriques des films. En effet, les films semblent plus sensibles aux effets de compression avec un potentiel électrique mesuré pour des forces faibles (11 - 5 mV à 0.4 N avec MAA contre 1 - 0.5 mV à 0.4 N sans MAA). De plus, la mesure effectuée à pH 6.5 met en avant l'importance des groupements carboxylates ionisés issus des unités MAA (50 % de groupement MAA ionisé) sur la sensibilité des films de microgels. (Cf. Tableau 2. *Effet de la composition en MAA)*
- Le pH de la solution de 5.5 à 7.5 sur les films de microgels de même composition ne semble pas modifier la valeur de potentiel électrique des films mais lorsque le pH de solution est augmenté, la perte du potentiel électrique face aux répétitions de compression semble ralentie. En effet à pH 5.5, la répétition de la compression fait chuter le potentiel électrique jusqu'à 2 mV alors qu'à un pH plus élevé, le potentiel chute jusqu'à 5.4 mV Ceci est probablement dû à l'augmentation de la proportion des fonctions carboxylate ionisées (%COO_{pH5.5} = 0 % ; %COO_{pH6.5} = 50 % ; %COO_{pH 7.5} = 75 %), augmentant la capacité de polarisation des microgels qui composent le film. Les films sont alors plus sensibles lorsque le pH est augmenté. (Cf. Tableau 2. *Effet du pH)*

### Propriétés optiques des microgels

Outre les propriétés mécano-électriques, les films de microgels se distinguent par leurs propriétés optiques, liées à la capacité de ces films à diffracter la lumière. Une disparité est observée en fonction de la composition des films :

### Films microgels P(MEO₂MA-co-OEGMA-co-MAA)

Lors du séchage d'une dispersion colloïdale de microgels sans nanoparticules, les films formés sont transparents à l'état sec et iridescent à l'état humide (Figure 7). Il semble que lors du gonflement des microgels le diamètre et la distance entre les particules favorisent une diffraction de la lumière dans le visible, cette diffraction est mise en évidence par l'observation de cristaux photoniques.

## Revendications

1. Produit cosmétique ou pharmaceutique contenant des microgels et au moins un composé choisi dans le groupe constitué par les agents tensio-actifs, les huiles, les produits biologiquement actifs, les pigments et les colorants, les microgels étant sous forme de particules susceptibles d'être obtenues par un procédé de copolymérisation en phase aqueuse des trois monomères suivants répartis de façon uniforme sur l'ensemble du volume des particules:
- le di(éthylène glycol) méthyl éther méthacrylate (M(EO)₂MA),
- un oligo(éthylène glycol) méthyl éther méthacrylate (M(EO)ₙMA) n étant un entier allant 3 à 12,
- un monomère de formule CR₁R₂=CR₃R₄ dans laquelle R₁, R₂, R₃ et R₄ représentent un hydrogène, un halogène ou un groupement hydrocarboné, à la condition qu'au moins un des quatre groupements comprenne un groupement -COOH ou -COO⁻M⁺, tel que M⁺ représente un cation,
en présence d'un agent réticulant,
les microgels ne comprenant pas des nanoparticules inorganiques.

2. Produit cosmétique ou pharmaceutique selon la revendication 1, **caractérisé en ce que** M(EO)₂MA représente 50% à 90% en moles du nombre total de moles des trois monomères, M(EO)ₙMA représente 10% à 50% en moles du nombre total de moles des trois monomères et le monomère de formule CR₁R₂=CR₃R₄ représente 0,1% à 20 % en moles du nombre total de moles des trois monomères.

3. Produit cosmétique ou pharmaceutique selon l'une des revendications précédentes **caractérisé en ce que** le monomère de formule CR₁R₂=CR₃R₄ est l'acide méthacrylique.

4. Produit cosmétique ou pharmaceutique selon l'une des revendications précédentes, **caractérisé en ce que** l'agent de réticulation est un oligo(éthylène glycol) diacrylate (OEGDA) comprenant de 1 à 10 motifs éthylène glycol.

5. Produit cosmétique ou pharmaceutique selon l'une des revendications 1 à 4, **caractérisé en ce que** le procédé comprend une étape de mise en contact en phase aqueuse - en présence d'un agent réticulant - des trois monomères à une température comprise entre 40°C et 90°C.

6. Produit cosmétique ou pharmaceutique selon l'une des revendications précédentes, **caractérisé en ce que** les microgels ne comprennent pas des molécules organiques.

## Patentansprüche

1. Kosmetisches oder pharmazeutisches Produkt, das Mikrogele und mindestens eine Verbindung enthält, die ausgewählt ist aus der Gruppe, bestehend aus Tensiden, Ölen, biologisch aktiven Produkten, Pigmenten und Farbstoffen, wobei die Mikrogele in Form von Teilchen sind, die durch ein Copolymerisationsverfahren in wässriger Phase der folgenden drei Monomere erlangt werden können, die gleichförmig über das gesamte Volumen der Partikel verteilt sind:
- di(Ethylenglycol)methylethermethacrylat (M(EO)₂MA),
- ein Oligo(ethylenglycol)methylethermethacrylat (M(EO)ₙ MA), wobei n eine ganze Zahl von 3 bis 12 ist,
- ein Monomer von Formel CR₁R₂=CR₃R₄, wobei R₁, R₂, R₃ und R₄ Wasserstoff, Halogen oder eine Kohlenwasserstoffgruppierung darstellen, unter der Bedingung, dass mindestens eine der vier Gruppierungen eine Gruppierung -COOH oder -COO-M⁺ umfasst, sodass M⁺ ein Kation darstellt, in
Anwesenheit eines Vernetzungsmittels,
wobei die Mikrogele keine anorganischen Nanopartikel umfassen.

2. Kosmetisches oder pharmazeutisches Produkt nach Anspruch 1, **dadurch gekennzeichnet, dass** M(EO)₂MA 50 bis 90 Mol-% der Gesamtmolzahl der drei Monomere darstellt, M(EO)ₙ MA 10 bis 50 Mol-% der Gesamtmolzahl der drei Monomere darstellt und das Monomer von Formel CR₁R₂=CR₃R₄ 0,1 bis 20 Mol-% der Gesamtmolzahl der drei Monomere darstellt.

3. Kosmetisches oder pharmazeutisches Produkt nach einem der vorherigen Ansprüche, **dadurch gekennzeichnet, dass** das Monomer von Formel CR₁R₂=CR₃R₄ Methacrylsäure ist.

4. Kosmetisches oder pharmazeutisches Produkt nach einem der vorherigen Ansprüche, **dadurch gekennzeichnet, dass** das Vernetzungsmittel ein Oligo(ethylenglykol)diacrylat (OEGDA) ist, umfassend 1 bis 10 Ethylenglykoleinheiten.

5. Kosmetisches oder pharmazeutisches Produkt nach einem der Ansprüche 1 bis 4, **dadurch gekennzeichnet, dass** das Verfahren einen Schritt eines Inkontaktbringens in wässriger Phase - in Anwesenheit eines Vernetzungsmittels - die drei Monomere bei einer Temperatur zwischen 40 °C und 90 °C umfasst.

6. Kosmetisches oder pharmazeutisches Produkt nach einem der vorherigen Ansprüche, **dadurch gekennzeichnet, dass** die Mikrogele keine organischen Moleküle umfassen.

## Claims

1. A cosmetic or pharmaceutical product containing microgels and at least one compound selected from the group consisting of surface active agents, oils, biologically active products, pigments and dyes,
wherein the microgels are in the form of particles obtainable by an aqueous phase copolymerization process of the following three monomers uniformly distributed over the entire volume of the particles:
- di(ethylene glycol) methyl ether methacrylate (M(EO)₂MA),
- an oligo(ethylene glycol) methyl ether methacrylate (M(EO)ₙMA) n being an integer ranging from 3 to 12,
- a monomer of formula CR₁R₂=CR₃R₄ wherein R₁, R₂, R₃ and R₄ represent a hydrogen, a halogen or a hydrocarbon group, provided that at least one of the four groups comprises a -COOH or -COO-M⁺ group, wherein M⁺ represents a cation,
in the presence of a crosslinking agent,
wherein the microgels do not comprise inorganic nanoparticles.

2. The cosmetic or pharmaceutical product according to claim 1, **characterized in that** M(EO)₂MA represents 50 mol% to 90 mol% of the total number of moles of the three monomers, M(EO)ₙMA accounts for 10 mol% to 50 mol% of the total number of moles of the three monomers and the monomer of formula CR₁R₂=CR₃R₄ accounts for 0.1 mol% to 20 mol% of the total number of moles of the three monomers.

3. The cosmetic or pharmaceutical product according to any one of the preceding claims, **characterized in that** the monomer of formula CR₁R₂=CR₃R₄ is methacrylic acid.

4. The cosmetic or pharmaceutical product according to any one of the preceding claims, **characterized in that** the crosslinking agent is an oligo(ethylene glycol) diacrylate (OEGDA) comprising from 1 to 10 ethylene glycol units.

5. The cosmetic or pharmaceutical product according to any one of claims 1 to 4, **characterized in that** the process comprises a step of contacting the three monomers in aqueous phase - in the presence of a crosslinking agent - at a temperature between 40°C and 90°C.

6. The cosmetic or pharmaceutical product according to any one of the preceding claims, **characterized in that** the microgels do not comprise organic molecules.
